# EUROPÄISCHE PATENTSCHRIFT

(11) **EP 3 242 102 B1**
(45) Veröffentlichungstag und Bekanntmachung des Hinweises auf die Patenterteilung: **07.11.2018**
(21) Anmeldenummer: 16167946.9
(22) Anmeldetag: 02.05.2016
(51) Int. Cl.: F28C 3/08, A47L 15/42, A61L 2/07, D06F 39/00

(54) **VERFAHREN ZUM ABKÜHLEN VON ABLUFT EINES REINIGUNGSGERÄTS, DAMPFKONDENSATOR UND REINIGUNGSGERÄT**
METHOD FOR COOLING EXHAUST AIR OF A CLEANING DEVICE, VAPOUR CONDENSER, AND CLEANING DEVICE
PROCEDE DE REFROIDISSEMENT D'AIR EVACUE D'UN DISPOSITIF DE NETTOYAGE, CONDENSATEUR DE VAPEUR ET APPAREIL DE NETTOYAGE

(43) Veröffentlichungstag der Anmeldung: 08.11.2017
(73) Patentinhaber: MELAG Medizintechnik oHG, 10829 Berlin (DE)
(72) Erfinder: HALBICH, Johannes, 10405 Berlin (DE); WENDTLAND, Mario, 14974 Ludwigsfelde (DE); FULDA, Felix, 10961 Berlin (DE)
(74) Vertreter: Maikowski & Ninnemann Patentanwälte Partnerschaft mbB

(56) Entgegenhaltungen:
- WO-A1-2005/037330
- WO-A1-2010/108887
- US-A- 1 458 697

## Beschreibung

Die Erfindung betrifft ein Verfahren zum Abkühlen von Abluft eines Reinigungsgeräts gemäß dem Oberbegriff des Anspruchs 1, einen zur Durchführung eines derartigen Verfahrens geeigneten Dampfkondensator gemäß dem Oberbegriff des Anspruchs 10 sowie ein Reinigungsgerät mit einem solchen Dampfkondensator gemäß dem Oberbegriff des Anspruchs 14.

Bei Reinigungsgeräten, wie etwa Reinigungs- und Desinfektionsgeräten, die häufig in Arztpraxen eingesetzt werden, aber auch bei Haushaltsgeschirrspülmaschinen, entsteht in einer Reinigungskammer eine große Menge feucht-warmer Luft, die aus dem Reinigungsgerät herausgeführt werden muss, ohne ein gesundheitliches Risiko für einen Benutzer durch mögliche Verbrühungen darzustellen. Daher wird die feucht-warme Luft regelmäßig gekühlt, bevor sie aus einem Reinigungsgerät herausgeführt wird. Dies ist insbesondere bei Reinigungs- und Desinfektionsgeräten, die aufgrund der durchzuführenden thermischen Desinfektion bei höheren Temperaturen arbeiten als "einfache" Reinigungsgeräte, der Fall.

Zur Abkühlung der feucht-warmen Luft werden regelmäßig Kondensatoren, sogenannte Dampfkondensatoren, eingesetzt. Die DE 10 2009 013 662 A1 beschreibt einen solchen Dampfkondensator, bei dem eine Kondensation des Dampfs in der Abluft eines Reinigungsgeräts und eine Abkühlung dieser Abluft durch Vermischung der Abluft mit einem Sprühnebel aus Wasser erreicht wird. Dabei kann innerhalb des Dampfkondensators eine Dampfsperre aufgebaut werden, um einen verlängerten Strömungsbereich für die abzukühlende Abluft zu definieren und so - neben der Kontaktierung mit feinsten Wassertröpfchen - eine weitere Abkühlung der Abluft zu erreichen.

Die EP 1 197 592 A2 beschreibt ein Kombinationsgerät aus einer Waschmaschine und einem Wäschetrockner. Dabei wird ein Standardkondensator eingesetzt, in dem Wasserdampf kondensiert wird. Dieser Kondensator wird zur besseren Wärmeübertragung mit Wasser gekühlt.

Die EP 1 673 111 B1 beschreibt ein Verfahren zur Kühlung von Reinigungsgut in Reinigungs-und Desinfektionsautomaten, bei dem die Abluft aus einer Reinigungskammer über eine separate Abluftleitung in eine Abwasserleitung geführt wird. Dabei wird durch konstruktive Vorgaben sichergestellt, dass die Abluft nicht mit Wasser, welches in der Abwasserleitung steht, in Kontakt kommt, sondern an diesem Wasser vorbeigeführt wird.

Eine vergleichbare Lösung ist auch aus der EP 2 120 672 B1 bekannt, die einen energieoptimierten Reinigungsautomaten beschreibt.

Der vorliegenden Erfindung liegt die Aufgabe zugrunde, ein Verfahren zum Abkühlen von Abluft eines Reinigungsgeräts anzugeben, das schneller und ressourcenschonender als aus dem Stand der Technik bekannte Abkühlungsverfahren durchgeführt werden kann. Ferner soll ein zur Durchführung eines derartigen Verfahrens geeignetes Gerät bereitgestellt werden.

Diese Aufgabe wird mit einem Verfahren zum Abkühlen von Abluft eines Reinigungsgeräts mit den Merkmalen des Anspruchs 1 gelöst. Dieses Verfahren kennzeichnet sich durch zwei Schritte. In einem ersten Schritt wird ein erstes Volumen Wasser in einen Dampfkondensator eines Reinigungsgeräts eingeleitet. Durch dieses Wasser wird eine Dampfsperre zwischen einem Ablufteinlass in den Dampfkondensator und einem Abluftauslass aus dem Dampfkondensator aufgebaut. Der Begriff "Dampfsperre" bezeichnet dabei ein Hindernis, welches von Wasserdampf oder feucht-warmer Abluft unter Normaldruck nicht überwunden werden kann. Vielmehr ist die Erzeugung einer Druckdifferenz zwischen einer ersten Seite der Dampfsperre und einer zweiten Seite der Dampfsperre erforderlich, um ein Überwinden der Dampfsperre für Wasserdampf bzw. Abluft eines Reinigungsgeräts zu ermöglichen.

In einem zweiten Verfahrensschritt wird Abluft aus einer Reinigungskammer des Reinigungsgeräts durch die aus dem Wasser gebildete Dampfsperre zum Abluftauslass des Dampfkondensators geleitet. Dabei tritt die Abluft in direkten Kontakt mit dem Wasser. Das Leiten ist ein aktives Leiten, welches mittels einer Druckdifferenz zwischen der ersten Seite der Dampfsperre und der zweiten Seite der Dampfsperre bewerkstelligt wird.

Durch ein derartiges aktives Leiten von Abluft durch die aus dem Wasser gebildete Dampfsperre werden überraschenderweise eine deutlich bessere Abkühlung und eine deutlich schnellere Entfeuchtung der Abluft erreicht. Während beispielsweise das aus der DE 10 2009 013 662 A1 bekannte Verfahren darauf abzielte, durch ein feines Versprühen von Wasser eine möglichst große Oberfläche zur Kondensation von Wasserdampf und zum Abkühlen von Abluft bereitzustellen, wurde durch die Erfinder der vorliegenden Erfindung überraschenderweise erkannt, dass eine derartige feine Verteilung von Wasser und das Bereitstellen einer großen Wasseroberfläche nicht maßgeblich für eine schnelle Abkühlung und gute Dampfkondensation ist. Vielmehr können durch das erfindungsgemäß beanspruchte Verfahren eine schnellere Abluftabkühlung und insbesondere eine schnellere Abluftentfeuchtung erfolgen. Wie gezeigt werden konnte, beruht dies auf einem zeitgleichen Kontakt der abzukühlenden Abluft mit einer größeren Wassermenge als bei dem aus dem Stand der Technik bekannten Sprühverfahren. Gleichzeitig ist die Gesamtwassermenge, die zur Abluftkühlung benötigt wird, gegenüber dem Prüfverfahren jedoch deutlich reduziert. Damit beruht die vorliegende Erfindung auf der zunächst widersinnig erscheinenden Erkenntnis, statt einer großen Wasseroberfläche und einem großen Wasservolumen ein kompaktes, geringeres Wasservolumen zur Abkühlung einzusetzen.

Wie im Zusammenhang mit einem Ausführungsbeispiel gezeigt werden wird, konnte der Wasserverbrauch gegenüber dem aus dem Stand der Technik bekannten Sprühverfahren mehr als halbiert werden. Gleichzeitig konnte die zur Abkühlung der Abluft benötigte Zeit auf deutlich weniger als ein Drittel der gemäß dem aus dem Stand der Technik bekannten Verfahren benötigten Zeit reduziert werden.

Gemäß einer Variante wird eine Druckdifferenz zwischen der ersten Seite der Dampfsperre und der zweiten Seite der Dampfsperre durch ein Gebläse erzeugt, welches die feucht-warme Abluft aus der Reinigungskammer des Reinigungsgeräts heraus saugt oder drückt. Der durch dieses Gebläse erzeugte Überdruck bzw. Unterdruck sorgt dann für ein Leiten der Abluft durch die Dampfsperre hindurch, wobei die Abluft in Form von Blasen in der Dampfsperre aufsteigt. Beim Durchleiten gibt die Abluft einen Teil ihrer thermischen Energie an das Wasser der Dampfsperre ab. Dieses Wasser kann daher auch als Kühlwasser bezeichnet werden. Gleichzeitig erfolgt eine Kondensation von in der Abluft enthaltenem Wasserdampf. Dies führt letztlich zu einer Erwärmung des Kühlwassers, wobei die Geschwindigkeit der Erwärmung von dem Verhältnis zwischen dem Volumen der abzukühlenden Abluft und dem ersten Volumen Wasser abhängt.

Das erste Volumen Wasser kann beispielsweise ein Volumen in einem Bereich von 300 ml bis 3 I, insbesondere 500 ml bis 2,5 I, insbesondere 600 ml bis 2,0 I, insbesondere 700 ml bis 1,5 I, insbesondere 800 ml bis 1,3 I, insbesondere 900 ml bis 1,2 I betragen.

Gemäß einer Variante wird das als Dampfsperre in dem Dampfkondensator befindliche Wasser entfernt, nachdem ein definiertes Volumen Abluft durch das Wasser hindurch geführt wurde. Alternativ kann das Wasser auch entfernt werden, wenn es eine bestimmte, vorgebbare Temperatur erreicht hat. Beispielsweise kann ein Schwellenwert vorgegeben werden, bei dessen Überschreiten eine weitere effektive Abluftkühlung nicht mehr möglich ist, sodass sich ein Entfernen des ersten Volumens Wasser anbietet. Alternativ kann auch die Temperatur der Abluft gemessen werden. Überschreitet die Temperatur der Abluft einen bestimmten Schwellenwert, bei dem Verletzungen eines Benutzers durch heiße austretende Abluft nicht sicher ausgeschlossen werden können, ist die Fähigkeit des in dem Dampfkondensator enthaltenen Wassers zur weiteren Abluftabkühlung offenbar erschöpft. In diesem Fall sollte das Wasser aus dem Dampfkondensator abgelassen werden.

Das Entfernen bzw. Ablassen des Wassers aus dem Dampfkondensator kann in einer Variante durch ein Abpumpen erfolgen. Alternativ kann das Wasser auch unter Ausnutzung der Schwerkraft abgelassen werden.

Gemäß einer Variante wird, nachdem das erste Volumen Wasser aus dem Dampfkondensator entfernt wurde, ein zweites Volumen Wasser in den Dampfkondensator eingeleitet. Dieses zweite Volumen Wasser dient abermals - wie das erste Volumen Wasser - zum Aufbau einer Dampfsperre zwischen dem Abluftauslass des Dampfkondensators und dem Ablufteinlass in den Dampfkondensator. Nachdem die Dampfsperre aufgebaut ist, wird abermals Abluft aktiv aus der Reinigungskammer des Reinigungsgerätes durch das Wasser hindurch zum Abluftauslass geleitet. Dabei tritt die Abluft - wie oben beschrieben - in direkten Kontakt mit dem Wasser, welches die Dampfsperre bildet.

Grundsätzlich kann sich das Volumen des zweiten Volumens Wasser von dem Volumen des ersten Volumens Wasser unterscheiden. Dabei kann es größer oder kleiner als das erste Volumen Wasser sein. In einer Variante sind die Volumina des ersten Volumens Wasser und des zweiten Volumens Wasser jedoch im Wesentlichen gleich.

Die vorstehend erläuterten Schritte des Aufbaus einer Dampfsperre mittels Wasser, des Leitens von Abluft durch das Wasser und des Ablassen des Wassers können beliebig oft wiederholt werden, um eine effektive Abluftabkühlung zu erreichen. Dabei muss das zur Abluftabkühlung eingesetzte Wasser umso öfter ausgetauscht werden, je geringer das eingesetzte Wasservolumen ist.

Gemäß einer Variante wird das zweite Volumen Wasser unter vergleichbaren Voraussetzungen aus dem Dammkondensator entfernt wie das erste Volumen Wasser. Die zur Entscheidung, ob das zweite Volumen Wasser aus dem Dampfkondensator entfernt werden soll, heranziehbaren Kriterien können folglich ein durch das Wasser durchgeleitete Volumen der abzukühlenden Abluft, die Temperatur des Kühlwassers oder die Temperatur der Abluft, nachdem sie das Kühlwasser passiert hat, sein. Diesbezüglich wird auf die obigen Erläuterungen hinsichtlich der möglichen Definition eines Schwellenwertes in Bezug auf die einzelnen Kriterien verwiesen.

Gemäß einer Variante wird, nachdem ein vorheriges Volumen Wasser aus dem Dampfkondensator entfernt wurde, ein drittes Volumen Wasser in den Dampfkondensator eingeleitet. Dabei dient das dritte Volumen Wasser dazu, eine Dampfsperre zwischen einem Ablufteinlass in den Dampfkondensator und einem Wasserzulauf aus dem Dampfkondensator in die Reinigungskammer des Reinigungsgerätes herzustellen. Das Volumen des dritten Volumens Wasser unterscheidet sich dabei von dem Volumen des zur Herstellung einer Dampfsperre zwischen dem Ablufteinlass in den Dampfkondensator und dem Abluftauslass aus dem Dampfkondensator benötigten Wassers. Regelmäßig ist das dritte Volumen kleiner als die zuvor eingesetzten Volumina. Insofern ist das dritte Volumen nicht geeignet, eine Dampfsperre zwischen dem Ablufteinlass in den Dampfkondensator und dem Abluftauslass aus dem Dampfkondensator aufzubauen. Folglich kann in den Dampfkondensator eintretende Abluft dann, wenn das dritte Volumen Wasser im Dampfkondensator vorhanden ist, den Dampfkondensator ohne Durchströmen des dritten Volumens Wasser passieren.

Dieser Verfahrensschritt ist insbesondere dann sinnvoll, wenn bereits eine signifikante Abluftabkühlung und Ablufttrocknung erfolgt ist, sodass weitere Abluft aus der Reinigungskammer des Reinigungsgeräts ohne direkten Wasserkontakt durch den Dampfkondensator hindurch und aus dem Dampfkondensator herausgeführt werden kann. Diese Variante bietet sich insbesondere dann an, wenn der Dampfkondensator mit eingebautem Wasserzulauf für ein mit dem Dampfkondensator verbundenes Reinigungsgerät ausgestattet ist. Denn dann wird durch die mittels des dritten Volumens Wasser gebildete Dampfsperre ein Kontakt zwischen dem Dampf und dem Wasserzulauf effektiv vermieden. Dies kann aus Gründen der Verhinderung einer etwaigen Kontamination des Wasserzulaufs sinnvoll und/oder durch behördliche Vorgaben vorgeschrieben sein.

Das dritte Volumen Wasser kann beispielsweise ein Volumen in einem Bereich von 10 ml bis 250 ml, insbesondere von 20 ml bis 200 ml, insbesondere von 30 ml bis 150 ml, insbesondere von 40 ml bis 100 ml, insbesondere von 50 ml bis 90 ml und ganz besonders von 60 ml bis 80 ml aufweisen.

In einer Variante wird die Abluft in nacheinander folgenden Pulsen durch das Kühlwasser hindurch zum Abluftauslass geleitet. Durch ein derartiges gepulstes Durchleiten der Abluft durch das Wasser kann eine zu schnelle Erwärmung des Wassers vermieden werden. Ein gepulstes Durchleiten des Wassers ist insbesondere dann sinnvoll, wenn das zur Verfügung stehende Volumen im Verhältnis zur durchzuführenden Abluft gering ist. Beispielsweise bietet sich ein gepulstes Durchleiten der Abluft bei einem Wasservolumen von weniger als 1,5 I, insbesondere von weniger als 1,2 I, insbesondere von weniger als 1 I, insbesondere von weniger als 0,8 I an.

Grundsätzlich ist es in einer anderen Variante jedoch auch möglich, selbst bei einem geringen Volumen des Kühlwassers die abzukühlende Abluft kontinuierlich durch das Wasser durchzuleiten. Ferner bietet sich ein kontinuierliches Durchleiten der Abluft dann an, wenn das Volumen des die Dampfsperre bildenden Kühlwassers 1,5 I oder mehr, insbesondere 1,7 I oder mehr, insbesondere 2 I oder mehr, insbesondere 2,5 I oder mehr beträgt. Geeignete Obergrenzen des Volumens des die Dampfsperre bildenden Kühlwassers sind beispielsweise 6 I, insbesondere 5l, insbesondere 4 I, insbesondere 3 I. Aus den vorgenannten beispielhaften Unter- und Obergrenzen des Volumens können beliebige Intervalle gebildet werden.

Wenn die Abluft gepulst durch das Kühlwasser geleitet wird, bietet es sich an, zunächst 1, 2, 3, 4, 5, 6, 7, 8, 9 oder 10 Pulse zu verwenden. Insbesondere sind 2, 3, 4 oder 5 Pulse, insbesondere 3, 4 oder 5 Pulse, insbesondere 3 oder 4 Pulse und ganz besonders 3 Pulse geeignet, um für eine effektive Abluftabkühlung in dem ersten Volumen Wasser zu sorgen.

Wenn das erste Volumen Wasser durch das zweite Volumen Wasser ausgetauscht wird, bietet es sich an, mit 5, 6, 7, 8, 9, 10, 11, 12, 13, 14, 15, 16, 17, 18, 19, oder 20 Pulsen für ein gepulstes Durchleiten der Abluft durch das zweite Volumen Wasser zu arbeiten. Insbesondere sind 10, 11, 12, 13, 14, 15, 16, 17 oder 18 Pulse, insbesondere 11, 12, 13, 14, 15, 16 oder 17 Pulse, insbesondere 12, 13, 14, 15 oder 16 Pulse, insbesondere 13, 14 oder 15 Pulse, insbesondere 13 oder 14 Pulse und ganz besonders 13 Pulse geeignet, um für eine effektive Abluftabkühlung in dem zweiten Volumen Wasser zu sorgen.

Wenn die Abluft mit Druck durch das die Dampfsperre bildende Wasser geleitet wird, kann es zu einer Bildung und zu einem nachfolgenden Platzen großer Abluftblasen und einer damit einhergehenden Geräuschentwicklung kommen. Gemäß einer Variante wird die Abluft daher durch eine Verteileinrichtung geführt, und zwar entweder bevor sie mit dem Kühlwasser in Kontakt kommt oder während sie sich bereits im Kühlwasser befindet. Die Verteileinrichtung sorgt für eine gleichmäßige Verteilung der Abluft über eine definierte Fläche. Beispielsweise kann die Verteileinrichtung als ein Filter oder ein Lochblech ausgeführt sein. Dann steigt die Abluft in kleineren Blasen weitgehend homogener Größe in dem die Dampfsperre ausbildenden Wasser auf. Dies führt zu einer Maximierung des Wärmeübergangs zwischen der Abluft und dem Wasser sowie zu einer Reduzierung der Geräuschentwicklung. Diese Variante bietet sich insbesondere dann an, wenn mit entsalztem oder deionisiertem Wasser oder VE-Wasser gearbeitet wird. Grundsätzlich eignet sich das vorliegend beschriebene Verfahren für den Einsatz der vorgenannten Wasserqualitäten, aber auch für den Einsatz von Leitungswasser unterschiedlichen Härtegrate.

Gemäß einer Variante wird das Volumen des in den Dampfkondensator eingeleiteten Wassers mittels eines geeigneten Sensors, wie etwa eines Durchflussmessers, bestimmt. Durch einen derartigen Sensor kann sehr genau bestimmt werden, welches Wasservolumen in den Dampfkondensator tatsächlich eingeleitet wurde. In einer weiteren Alternative wird die Einlaufzeit vorgegeben, während der das Wasser in den Dampfkondensator eingeleitet wird. Wenn die maximal mögliche Durchflussrate des Wassers am Wassereinlass bekannt ist und bei der maximal möglichen Durchflussrate gearbeitet wird, lässt sich über die Einlaufzeit problemlos die in den Dampfkondensator tatsächlich eingeleitete Wassermenge bestimmen.

Die Erfindung betrifft auch einen Dampfkondensator für ein Reinigungsgerät mit den Merkmalen des Anspruchs 10. Ein derartiger Dampfkondensator weist einen Strömungsbereich für Abluft aus einem Reinigungsgerät, einen an einem Ende des Strömungsbereichs angeordneten Abluftauslass und einen Wassereinlass auf. Durch den Wassereinlass kann Wasser in den Dampfkondensator eingebracht werden.

Der erfindungsgemäß beanspruchte Dampfkondensator zeichnet sich dadurch aus, dass der Strömungsbereich in einer spezifischen Art und Weise geformt ist. Dadurch muss die zum Abluftauslass strömende Abluft aus dem Reinigungsgerät einen Abschnitt des Strömungsbereichs durchströmen, der im bestimmungsgemäßen Betrieb des Dampfkondensators vollständig mit Wasser gefüllt ist. Das heißt, die Abluft wird zwangsweise durch eine aus Wasser gebildete Dampfsperre geführt (was, wie oben erläutert, nur durch eine entsprechende Luftdruckdifferenz zwischen der ersten Seite der Dampfsperre und der zweiten Seite der Dampfsperre möglich ist). Beim Durchströmen dieses Strömungsbereichsabschnitts tritt die Abluft zudem in direkten Kontakt mit dem Wasser. Der Dampfkondensator weist also keine Leitung auf, die die Dampfsperre überbrückt und durch die die Abluft strömen könnte.

Da eine Dampfsperre aus Wasser insbesondere in einem unteren Bereich des Dampfkondensators gut ausgebildet werden kann, wird die Abluft vorzugsweise in einem unteren Bereich des Dampfkondensators in diesen eingeleitet oder zu einem unteren Bereich des Dampfkonzertes geführt, um dann das als Dampfsperre fungierende Wasser und den weiteren Strömungsbereich des Dampfkondensators zu durchströmen.

Gemäß einer Variante ist im Strömungsbereich des Dampfkondensators eine Barriere angeordnet. Dabei sorgt die Barriere dafür, dass das Wasser, welches sich im bestimmungsgemäßen Betrieb des Dampfkondensators im Innern des Dampfkondensators befindet und die Dampfsperre bildet, nicht von einer ersten Seite der Barriere auf eine zweite Seite der Barriere gelangen kann. Demgegenüber kann die Abluft die Barriere überwinden, also von der ersten Seite der Barriere zu der zweiten Seite der Barriere gelangen. Der Begriff "nicht überwinden" bzw. "nicht gelangen" ist dabei derart auszulegen, dass er erfüllt ist, wenn 90 % oder mehr, insbesondere 95 % oder mehr, insbesondere 97 % oder mehr, insbesondere 98 % oder mehr, insbesondere 99 % oder mehr, insbesondere 99,5 % oder mehr des Wassers, das auf der ersten Seite der Barriere ist, nicht die zweite Seite der Barriere erreicht.

Die Barriere kann beispielsweise aus einem oder mehreren Prallblechen aufgebaut sein. Werden mehrere Prallbleche eingesetzt, ist es vorteilhaft, diese parallel zueinander anzuordnen. Dabei können die Prallbleche beispielsweise oberhalb des die Dampfsperre bildenden Wassers im Dampfkondensator angeordnet werden, sodass das Wasser dann, wenn Dampf durch es hindurch geleitet wird, gegen die Unterseite der jeweiligen Prallbleche gedrückt wird. Die erste Seite der Barriere wäre dabei die Unterseite des untersten Prallblechs, während die zweite Seite der Barriere die Oberseite des obersten Prallblechs wäre.

Da insbesondere das Prallblech oder die Prallbleche, die näher zu dem Wasser hin orientiert sind, teilweise von Wasser umspült werden können, ist es sinnvoll, die Prallbleche nicht genau horizontal, sondern leicht schräg anzuordnen, um für ein gutes Ablaufen des Wassers von den Oberseiten der Prallbleche zu sorgen.

Gemäß einer weiteren Variante weist der Dampfkondensator nicht nur einen Wassereinlass auf, mit dem Wasser in den Innenraum des Dampfkondensators geleitet werden kann, um eine Dampfsperre aufzubauen. Vielmehr ist der Dampfkondensator in dieser Variante auch mit einer Wasserversorgung für ein mit dem Dampfkondensator verbundenes Reinigungsgerät ausgestattet. Dann bietet es sich an, den Wassereinlass für den Dampfkondensator und die Wasserversorgung für das Reinigungsgerät in einem gemeinsamen Bauraum innerhalb des Dampfkondensators anzuordnen. Dieser Bauraum ist nicht dafür vorgesehen, von Wasserdampf durchströmt zu werden. Zum Aufbau der Dampfsperre ist es jedoch erforderlich, dass das Wasser aus diesem Bauraum über eine entsprechende Strömungsverbindung mit dem Strömungsbereich für die Abluft in Verbindung steht. Dabei ist diese Strömungsverbindung vorzugsweise derart ausgestaltet, dass sie bei Bedarf durch das Einlassen von Wasser für Gase undurchlässig im Sinne einer Dampfsperre ausgestaltet werden kann. Die Wasserversorgung für das Reinigungsgerät kann beispielsweise als freier Auslauf ausgestaltet sein.

Gemäß einer weiteren Variante ist in dem Abschnitt des Dampfkondensators, in dem im bestimmungsgemäßen Betrieb eine Dampfsperre ausgebildet ist, die von der Abluft durchströmt werden soll, eine Verteileinrichtung vorgesehen, die für eine gleichmäßige Verteilung der Abluft über eine definierte Fläche sorgt.

Wie bereits zuvor erwähnt, kann es sich bei einer derartigen Verteileinrichtung beispielsweise um einen Filter oder ein Lochblech handeln. Dabei sorgt die Verteileinrichtung für die Bildung feiner Abluftblasen innerhalb des Wassers, wodurch ein Wärmeübergang von der Abluft in das Wasser maximiert und eine Geräuschentwicklung reduziert wird. Die Verteileinrichtung ist dabei derart in dem vorgenannten Abschnitt des Dampfkonzertes angeordnet, dass die Abluft nicht an der Verteileinrichtung vorbeiströmen kann, sondern die Verteileinrichtung passieren muss. Dadurch erhöht sich zwar der Widerstand, den die Abluft überwinden muss, um durch den Dampfkondensator zu strömen. Durch das Aufbringen eines entsprechenden Drucks auf die Abluft - beispielsweise durch ein Gebläse - kann dieser zusätzliche Gegendruck jedoch leicht überwunden werden.

Die Erfindung betrifft auch ein Reinigungsgerät, das mit einem Dampfkondensator entsprechend den vorherigen Erläuterungen ausgestattet ist. Die Vorteile eines derartigen Reinigungsgeräts, das beispielsweise ein Reinigungs- und Desinfektionsgerät, insbesondere ein Reinigungs- und Desinfektionsgerät für medizinische und/oder zahnmedizinische Übertragungsinstrumente und Apparate, sein kann, ergeben sich unmittelbar aus den vorherigen Erläuterungen zum Dampfkondensator bzw. zum Verfahren zum Abkühlen der Abluft eines Reinigungsgeräts.

Insbesondere ist es durch eine effektive Abkühlung der Abluft des Reinigungsgeräts möglich, die Abluft in einem vorderen Bereich des Reinigungsgeräts in die Umgebung des Reinigungsgeräts abzulassen. Ein derartiges, nach vorne gerichtetes Austragen der Abluft aus dem Reinigungsgerät ist nur dann möglich, wenn die Abluft effektiv abgekühlt wurde. Denn andernfalls bestünde die Gefahr von Verletzungen für einen Benutzer. Ein nach vorne gerichtetes Austragen der Abluft ist gegenüber einem seitlichen oder nach hinten gerichteten Austragen der Abluft jedoch deutlich vorteilhafter. Denn üblicherweise sind Reinigungsgeräte in eine Gerätezeile eingebaut. Wird nun eine (immer noch Restfeuchte enthaltende) Abluft in einen seitlichen oder hinteren Bereich einer derartigen Gerätezeile abgegeben, kommt es zu einer Kondensation der Restfeuchtigkeit und einer nachfolgenden Schimmelbildung. Dies wird durch ein Austragen der Abluft nach vorne effektiv vermieden. Alternativ ist es auch möglich, den Abluftauslass des Dampfkondensators des Reinigungsgeräts an eine (dentale) Absauganlage anzuschließen, um ein Entweichen der Abluft in die Umgebung des Reinigungsgeräts effektiv zu vermeiden.

Bevorzugte Ausgestaltungen und Varianten des beschriebenen Verfahrens sind in analoger Weise auf den beschriebenen Dampfkondensator und das beschriebene Reinigungsgerät übertragbar, und umgekehrt. Dabei sind sämtliche der beschriebenen Ausführungsformen und Varianten in beliebiger Art und Weise miteinander kombinierbar.

Die vorliegende Erfindung wird nachfolgend anhand von Figuren und einem Ausführungsbeispielen näher erläutert. Es zeigen:
- Figur 1: eine Schnittansicht durch ein Ausführungsbeispiel eines Dampfkondensators in einem ersten Betriebszustand;
- Figur 2: eine Schnittansicht durch den Dampfkondensator der Figur 1 in einem zweiten Betriebszustand;
- Figur 3: eine Schnittansicht durch den Dampfkondensator der Figur 1 in einem dritten Betriebszustand;
- Figur 4: eine gegenüber der Figur 2 um 90° gedrehte seitliche Schnittansicht durch den Dampfkondensator der Figur 1 in dem in der Figur 2 dargestellten Betriebszustand; und
- Figur 5: eine isometrische Ansicht des in den Figuren 1 bis 4 dargestellten Dampfkondensators.

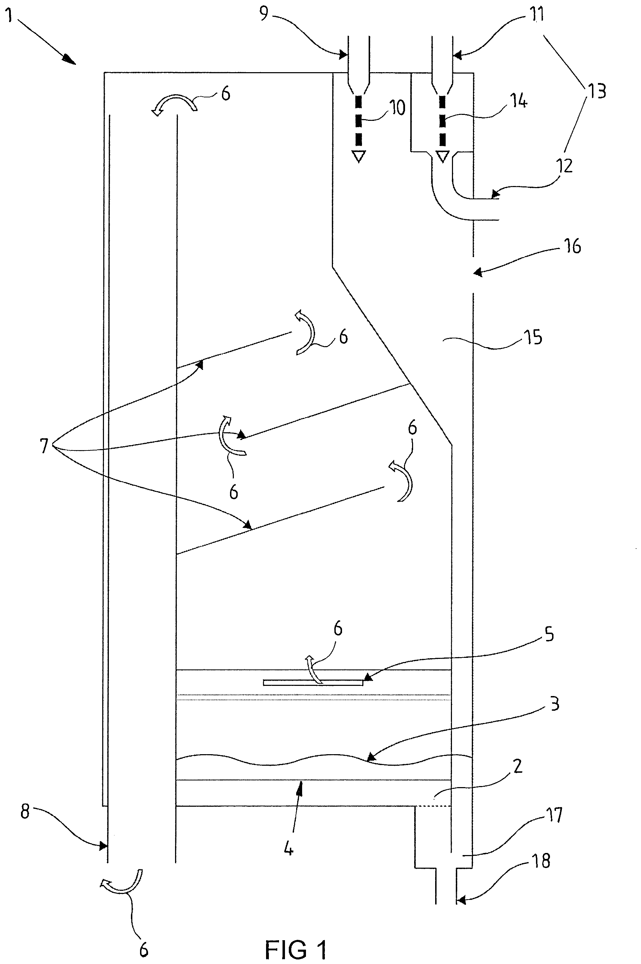

Die Figur 1 zeigt einen Dampfkondensator 1, der Teil eines Reinigungsgerätes ist, das in der Figur 1 nicht dargestellt ist. Das Reinigungsgerät ist als Reinigungs- und Desinfektionsgerät ausgestaltet. Die Figur 1 zeigt einem Betriebszustand des Dampfkondensators 1 während eines Reinigungsschritts bzw. eines Desinfektionsschritts des von dem Reinigungsgerät durchgeführten Reinigungsverfahrens. In diesem ersten Betriebszustand des Dampfkondensators ist ein unterer Bereich des Dampfkondensators mit Wasser 2 gefüllt.

Dabei befindet sich ein erster Wasserstand 3 des Wassers 2 oberhalb eines Ablufteinlasses 4, durch den Abluft aus dem Reinigungsgerät in den Dampfkondensator 1 grundsätzlich eintreten kann. Aufgrund des oberhalb des Ablufteinlasses 4 stehenden ersten Wasserstandes 3 ist es der Abluft jedoch nicht möglich, durch den Ablufteinlass 4 in den Dampfkondensator einzutreten. Vielmehr wirkt das Wasser 2 als Dampfsperre gegenüber der Abluft, die aus dem Reinigungsgerät zum Dampfkondensator hin entweicht.

Um für einen Druckausgleich zwischen einer Reinigungskammer des Reinigungsgeräts und dem Dampfkondensator 1 bzw. einer das Reinigungsgerät umgebenden Umgebung zu sorgen, weist der Dampfkondensator 1 einen Abluftbypass 5 auf, der oberhalb des ersten Wasserstands 3 angeordnet ist. Durch diesen Abluftbypass 5 kann Abluft 6 aus dem Reinigungsgerät in den Dampfkondensator eintreten, um mehrere Prallbleche 7 herum durch den Dampfkondensator 1 strömen und den Dampfkondensator 1 schließlich durch einen Abluftauslass 8 verlassen.

Dabei ist die Größe des Abluftbypasses 5 derart bemessen, dass nur geringe Volumina Abluft 6 durch den Abluftbypass 5 treten und letztlich durch den Abluftauslass 8 aus dem Dampfkondensator austreten können. Derartige Abluftvolumina kühlen sich auf dem Weg durch den Dampfkondensator hinreichend ab, sodass sie keine Gefährdung für einen Benutzer darstellen und keine Dampfwrasen an der Gerätefront sichtbar sind. So dient der Abluftbypass 5 - wie bereits oben angedeutet - insbesondere zu einem Druckausgleich zwischen der Reinigungskammer des Reinigungsgeräts und einer Umgebung des Reinigungsgeräts.

In diesem Betriebszustand des Dampfkondensators 1 sollen also keine größeren Volumina Abluft 6 aus dem Reinigungsgerät bzw. dem Dampfkondensator 1 herausgeführt werden.

Um das Wasser 2 in den unteren Bereich des Dampfkondensators 1 einzubringen, weist der Dampfkondensator 1 einen Kühlwasserzulauf 9 auf, der als Wassereinlass fungiert. Durch diesen Kühlwasserzulauf 9 kann Kühlwasser 10 in den Dampfkondensator 1 geleitet werden und - wie in der Figur 1 dargestellt - als Wasser 2 im unteren Bereich des Dampfkondensators 1 vorgehalten werden.

Der Dampfkondensator 1 weist ferner einen freien Auslauf 11 und einen Wassereinlass 12 zu einer Reinigungskammer des angeschlossenen Reinigungsgeräts auf. Der freie Auslauf 11 und der Wassereinlass 12 zur Reinigungskammer bilden beide zusammen eine Wasserversorgung 13 für das Reinigungsgerät. Dabei fließt Reinigungswasser 14 aus dem freien Auslauf 11 in den Wassereinlass 12 und von dort weiter zur Reinigungskammer des Reinigungsgeräts.

Der Kühlwasserzulauf 9 und die Wasserversorgung 13 sind in einem gemeinsamen Bauraum 15 des Dampfkondensators angeordnet. Auf diese Weise ist es möglich, den Kühlwasserzulauf 9 und die Wasserversorgung 13 räumlich dicht beieinander zu halten, was einen Anschluss des Kühlwasserzulaufs 9 und der Wasserversorgung 13 an eine externe Wasserleitung erleichtert.

Im Bauraum 15 ist zudem ein Überlauf 16 vorgesehen, der in einem Havariefall ein Austreten von Wasser aus dem gemeinsamen Bauraum 15 zum tiefsten Punkt des Reinigungsgeräts ermöglicht.

Das Kühlwasser 10 wird aus dem Kühlwasserzulauf 9 durch den gemeinsamen Bauraum 15 zu einem unteren Bereich des Dampfkondensators 1 geführt. In diesem unteren Bereich ist ein Kühlwassersiphon 17 ausgebildet. An einem unteren Ende des Kühlwassersiphons 17 ist zudem ein Kühlwasserablauf 18 vorgesehen, durch den das Wasser 2 aus dem Dampfkondensator 1 -beispielsweise durch Abpumpen -entfernt werden kann. In dem ersten Betriebszustand, der in der Figur 1 dargestellt ist, ist der Kühlwasserablauf 18 zunächst jedoch verschlossen, sodass das Wasser 2 im Dampfkondensator 1 stehen bleibt.

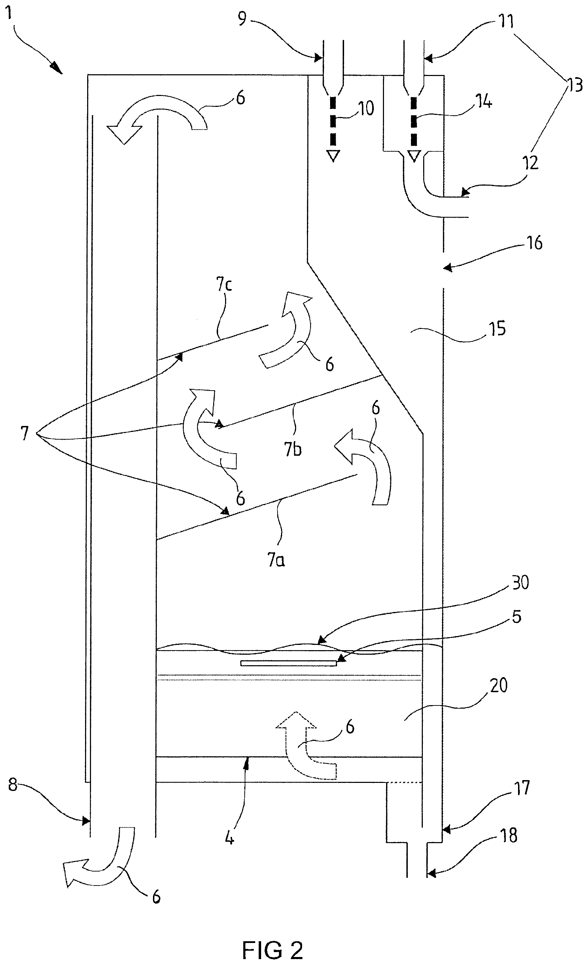

Die Figur 2 zeigt einen zweiten Betriebszustand des Dampfkondensators 1 der Figur 1. Dieser zweite Betriebszustand wird dann eingenommen, wenn aus dem mit dem Dampfkondensator verbundenen Reinigungsgerät Abluft in größerer Menge abgeführt und durch den Dampfkondensator 1 abgekühlt werden soll. Gleiche Elemente des Dampfkondensators 1 werden mit gleichen Bezugszeichen wie in der Figur 1 bezeichnet. Nachfolgend wird nur auf die Unterschiede zu dem in der Figur 1 dargestellten ersten Betriebszustand des Dampfkondensators 1 eingegangen.

Im zweiten Betriebszustand ist eine größere Menge Wasser 20 im unteren Bereich des Dampfkondensators 1 vorhanden. Diese größere Menge Wasser 20 kann auch als erstes Volumen Wasser bezeichnet werden und ist als Kühlwasser 10 durch den Kühlwasserzulauf 9 in den Dampfkondensator 1 eingebracht worden.

Der zweite Wasserstand 30 der größeren Wassermenge 20 überdeckt nun sowohl den Ablufteinlass 4 als auch den Abluftbypass 5. Dadurch ist im zweiten Betriebszustand kein Druckausgleich wie im ersten Betriebszustand zwischen der Reinigungskammer und einer Umgebung möglich. Dies ist auch nicht erforderlich, da im zweiten Betriebszustand ein Gebläse aktiviert wird, welches Abluft aus der Reinigungskammer durch den Dampfkondensator 1 drückt. Der durch das Gebläse aufgebaute Druck ist dabei so stark, dass die durch die größere Wassermenge 20 erzeugte Dampfsperre von der Abluft 6 überwunden werden kann und die Abluft 6 aus der Reinigungskammer durch den Ablufteinlass 4 in den Dampfkondensator 1 eintritt. Dabei durchströmt die Abluft 6 die größere Wassermenge 20 und kommt in direkten Kontakt mit dem Wasser. Anschließend strömt die Abluft 6 um die Prallbleche 7 herum und verlässt den Dampfkondensator 1 durch den Abluftauslass 8.

Wenn die Abluft 6 durch die größere Wassermenge 20 gedrückt wird, kommt es zu einer Blasenbildung in der größeren Wassermenge 20. Dadurch wird das Wasser nach oben gedrückt und prallt an das unterste Prallblech 7a und gegebenenfalls an die Unterseite des mittleren Prallblechs 7b. Durch die zueinander versetzte Anordnung der drei Prallbleche 7a, 7b und 7c wird jedoch effektiv vermieden, das Wasser der größeren Wassermenge 20 von der der Unterseite des untersten Prallblechs 7a zugewandten Seite zu der der Oberseite des obersten Prallblechs 7c zugewandten Seite des Dampfkondensators gelangt. Damit dienen die Prallbleche 7 als effektive Barriere gegen einen Wasseraustritt aus dem Dampfkondensator 1. Letztlich strömt das Wasser immer wieder zum unteren Bereich des Dampfkondensators 1 zurück, während die Abluft 6 nach ihrer Abkühlung durch die größere Wassermenge 20 aus dem Dampfkondensator 1 austreten kann.

Beim Austritt der Abluft 6 aus dem Dampfkondensator 1 ist die Abluft 6 dabei so weit abgekühlt, dass eine Verbrühung eines Benutzers ausgeschlossen werden kann, selbst wenn der Benutzer in direkten Kontakt mit der Abluft 6 kommt. Dafür sorgt ein Wärmeübergang aus der Abluft 6 in die größere Wassermenge 20. Beim direkten Kontakt zwischen der Abluft 6 unter größeren Wassermenge 20 kommt es darüber hinaus zu einer Kondensation von Wasserdampf, der in der Abluft 6 enthalten ist. Dies reduziert den Energiegehalt der Abluft 6 weiter.

Für eine effektive Abkühlung der Abluft 6 kann die größere Wassermenge 20 einmal oder mehrmals gegen eine vergleichbare neue Wassermenge ausgetauscht werden. Dazu wird die größere Wassermenge 20 durch den Kühlwasserablauf 18 abgelassen bzw. abgepumpt. Anschließend wird der Kühlwasserablauf 18 wieder verschlossen und neues Kühlwasser 10 durch den Kühlwasserzulauf 9 in den Dampfkondensator eingeleitet. Das Gebläse, welches die Abluft aus der Reinigungskammer zum Dampfkondensator 1 trägt, wird während des Ablaufs des Wassers aus dem Dampfkondensator 1 deaktiviert, sodass in dieser Zeit keine relevante Abluftmenge in den Dampfkondensator 1 eintritt. Erst wenn die durch die größere Wassermenge 20 aufgebaute Dampfsperre wieder hergestellt ist, wird das Gebläse erneut aktiviert, so das dann - wie oben dargestellt - Abluft 6 durch den Ablufteinlass 4 in den Dampfkondensator 1 eingetragen wird.

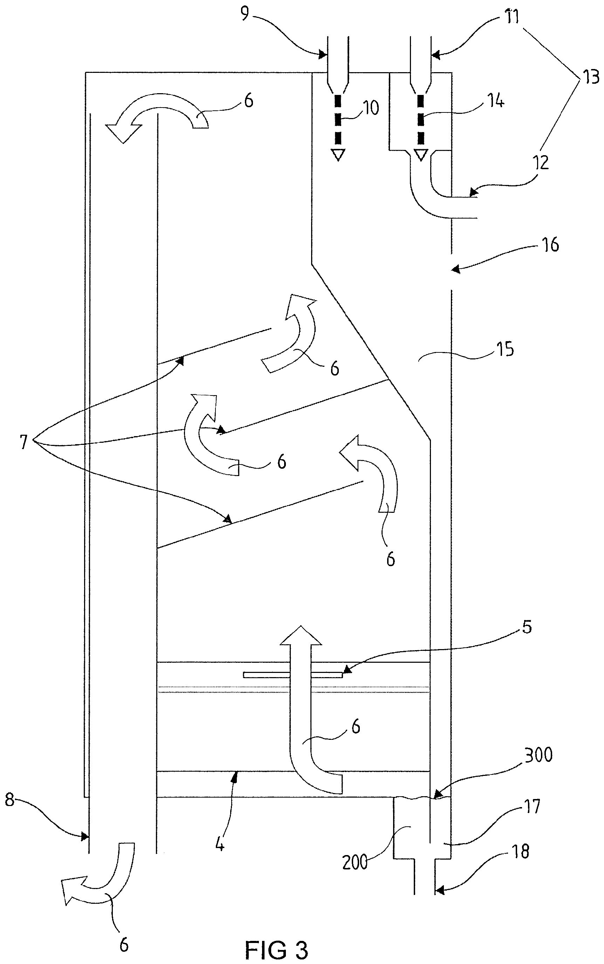

Die Figur 3 zeigt den aus den Figuren 1 und 2 bekannten Dampfkondensator 1 in einem dritten Betriebszustand. Abermals werden dieselben Bezugszeichen für dieselben Elemente des Dampfkondensators 1 verwendet, wobei wiederum nur Unterschiede zu den bisherigen Betriebszuständen erläutert werden.

Der Dampfkondensator 1 wird im dritten Betriebszustand dann betrieben, wenn die Abluft 6 aus der Reinigungskammer, mit der der Dampfkondensator 1 verbunden ist, bereits hinreichend abgekühlt ist und nun eine Trocknung des in der Reinigungskammer enthaltenen Reinigungsguts erfolgen soll. Zu diesem Zweck befindet sich im Dampfkondensator 1 nur noch eine geringere Wassermenge 200, die ein drittes Volumen Wasser darstellt. Ein dritter Wasserstand 300 der geringeren Wassermenge 200 liegt dabei unterhalb des Niveaus des Ablufteinlasses 4 und des Abluftbypasses 5. Damit bildet die geringere Wassermenge 200 lediglich eine Dampfsperre zwischen dem Ablufteinlass 4 und dem Kühlwasserzulauf 9 bzw. dem für den Havariefall vorgesehenen Überlauf 16. Die geringere Wassermenge 200 ist jedoch nicht geeignet, um eine Dampfsperre zwischen dem Ablufteinlass 4 und dem Abluftauslass 8 des Dampfkondensators 1 aufzubauen.

Wird nun das Gebläse des Reinigungsgeräts aktiviert, welches die Abluft aus der Reinigungskammer durch den Ablufteinlass 4 in den Dampfkondensator 1 einträgt, kann die Abluft 6 ungehindert durch den Ablufteinlass 4 durch den Dampfkondensator 1 strömen. Die Abluft 6 wird lediglich daran gehindert, den gemeinsamen Bauraum 15, in dem der Kühlwasserzulauf 9 und die Wasserversorgung 13 angeordnet sind, zu erreichen. Auf diese Weise wird die Abluft 6 durch den Dampfkondensator 1 geführt, ohne in direkten Kontakt mit Wasser bzw. Kühlwasser zu kommen. Dadurch ist die Geräuschentwicklung während dieses Trocknungsschritts geringer, als wenn durch einen Durchtritt der Abluft 6 durch Wasser eine Blasenbildung mit entsprechender Geräuschentwicklung auftritt. Gleichzeitig ist der Widerstand, den die Abluft 6 beim Durchströmen des Dampfkondensators 1 überwinden muss, geringer als im zweiten Betriebszustand, der in der Figur 2 dargestellt ist.

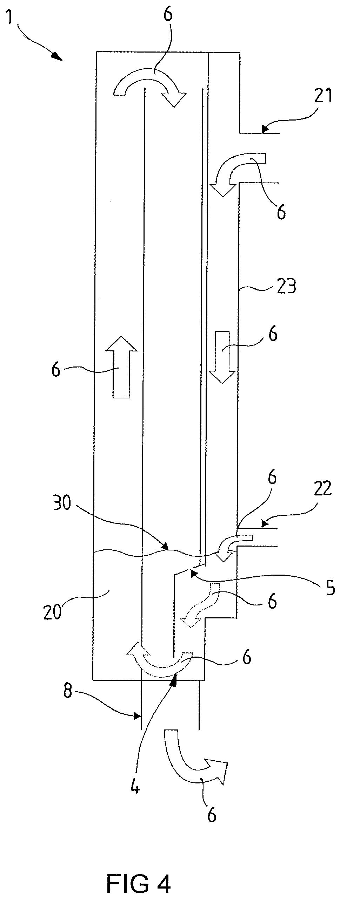

Die Figur 4 zeigt den in der Figur 2 bereits dargestellten zweiten Betriebszustand des Dampfkondensators 1 in einer weiteren Schnittansicht, die gegenüber der Schnittansicht der Figur 2 um 90° gedreht ist. Es werden wiederum dieselben Bezugszeichen für die bereits im Zusammenhang mit den Figuren 1 bis 3 erläuterten Elemente verwendet. Ferner wird auf die obigen Erläuterungen hinsichtlich der einzelnen Elemente verwiesen.

In dieser Ansicht sind ein erster Abluftanschluss 21 und ein zweiter Abluftanschluss 22 zu sehen, die den Dampfkondensator 1 mit der Reinigungskammer des Reinigungsgerätes verbinden. Durch den ersten Abluftanschluss 21 und den zweiten Abluftanschluss 22 sowie einen Abluftkanal 23, der den ersten Abluftanschluss 21 und den zweiten Abluftanschluss 22 miteinander verbindet, strömt die Abluft 6 aus der Reinigungskammer des Reinigungsgeräts zum Dampfkondensator 1. Dabei muss die Abluft 6 bereits kurz unterhalb des zweiten Abluftanschlusses 22 in die größere Wassermenge 20 eintreten, da der zweite Wasserstand 30 knapp unterhalb des zweiten Abluftanschlusses 22 liegt.

Die Abluft 6 durchströmt dann die größere Wassermenge 20 und tritt durch den Ablufteinlass 4 in denjenigen Bereich des Dampfkondensators 1 ein, der in der Schnittdarstellung der Figur 2 zu sehen ist. Dort strömt die Abluft 6 weiter durch die größere Wassermenge 20, umströmt nachfolgend die in der Figur 4 nicht zusehenden Prallbleche 7 und kann den Dampfkondensator 1 anschließend abgekühlt durch den Abluftauslass 8 verlassen.

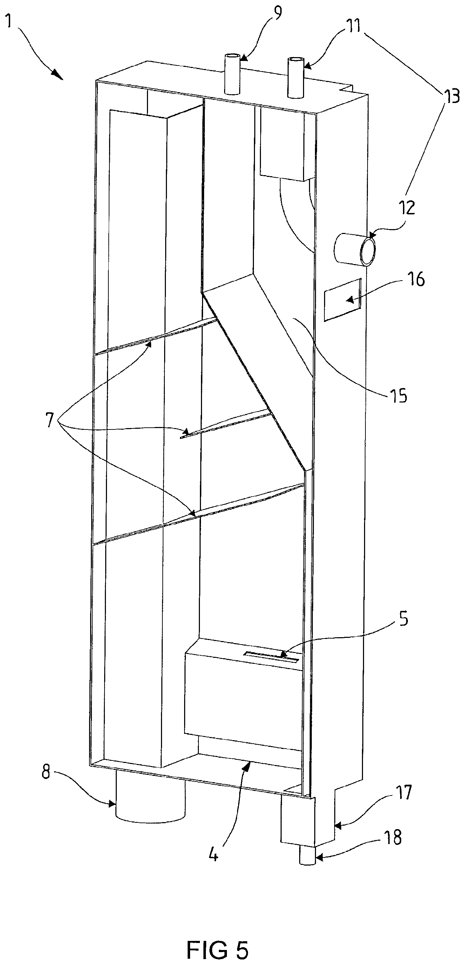

Die Figur 5 zeigt eine isometrische Ansicht des Dampfkondensators 1 ohne in den Dampfkondensator 1 eingelassenes Wasser. Diese Darstellung dient insbesondere dem besseren Verständnis der konstruktiven Ausgestaltung des Dampfkondensators 1. Es werden wiederum dieselben Bezugszeichen für die bereits im Zusammenhang mit den Figuren 1 bis 4 erläuterten Elemente verwendet. Ferner wird auf die obigen Erläuterungen hinsichtlich der einzelnen Elemente verwiesen.

### Ausführungsbeispiel

Der in den Figuren 1 bis 5 dargestellte Dampfkondensator wurde zur Abkühlung von Abluft aus einem Reinigungs- und Desinfektionsgerät eingesetzt. Dabei wurde mit 700 ml Kühlwasser als erstem Volumen Kühlwasser und 700 ml Kühlwasser als zweitem Volumen Kühlwasser gearbeitet. Ferner wurde die Abluft gepulst durch das Kühlwasser durchgeleitet. So erfolgte ein Durchleiten der Abluft für 2 Sekunden durch das Kühlwasser. Anschließend erfolgten 3 Sekunden Pause. Dieser Puls bzw. dieses Intervall wurde insgesamt dreimal durchgeführt. Anschließend wurde das erste Volumen Kühlwasser durch das zweite Volumen Kühlwasser ersetzt. Die Abluft wurde abermals für 2 Sekunden durch das zweite Volumen Kühlwasser geleitet. Anschließend erfolgte eine 3-sekündige Pause. Dieses Intervall bzw. dieser Puls wurde dreizehnmal durchgeführt. Anschließend wurde auch das zweite Volumen Kühlwasser abgepumpt.

Die aus dem Dampfkondensator austretende Abluft hatte beim ersten Abkühlschritt (mit dem ersten Volumen Kühlwasser) eine Temperatur von maximal 47 °C und beim zweiten Abkühlschritt (mit dem zweiten Volumen Kühlwasser) eine Temperatur von maximal 48,8 °C.

Insgesamt wurde während der Abkühlung ein Wasservolumen in Höhe von 1,13 l verbraucht (400 ml zu bereits im Gerät befindlichen 300 ml, die als Dampfsperre während eines zuvor durchgeführten thermischen Desinfektionsschritts eingesetzt wurden, für den ersten Abkühlschritt plus 700 ml für den zweiten Abkühlschritt plus 30 ml zum Aufbau einer Dampfsperre während des Trocknungsschritts).

Die bei dem Verfahren ermittelten Verbrauchsparameter wurden mit den Verbrauchsparametern verglichen, die im Universalprogramm mit dem in der DE 10 2009 013 662 A1 beschriebenen Dampfkondensator erhalten wurden (es wurde jeweils dasselbe Reinigungs- und Desinfektionsprogramm eines - abgesehen vom Dampfkondensator - baugleichen Reinigungs- und Desinfektionsgeräts durchgeführt).

In der nachfolgenden Tabelle sind die Verbrauchsparameter aufgelistet. Daraus ergibt sich, dass die für die Kühlung benötigte Zeit signifikant von 15 Minuten auf 3 Minuten und 50 Sekunden reduziert werden konnte. Davon entfielen 2 Minuten auf den Austausch des ersten Volumens Kühlwasser durch das zweite Volumen Kühlwasser. Hinsichtlich der bei der Trocknung vorliegenden Starttemperatur wurde gegenüber dem bislang durchgeführten Verfahren zwar eine Temperaturerhöhung von 11 °C beobachtet. Allerdings ist die zum Startzeitpunkt der Trocknung in der Reinigungskammer des Reinigungs- und Desinfektionsgeräts enthaltene Luft bei dem erfindungsgemäß beanspruchten Verfahren deutlich trockener, da eine deutlich stärkere Entfeuchtung der Luft während des Abkühlschritts erfolgt ist. Dadurch resultiert eine signifikant bessere Restverdunstung der noch in der Reinigungskammer enthaltenen Feuchtigkeit. Ferner wird eine Wrasenbildung effektiv vermieden.

Der Wasserverbrauch konnte von 2,5 I auf 1,13 I reduziert werden und ist damit weniger als halb so groß wie bei dem aus dem Stand der Technik bekannten Verfahren.

Insgesamt ergibt sich durch das erfindungsgemäß beanspruchte Verfahren damit eine signifikante Zeitreduzierung von mehr als 11 Minuten und ein um ca. 1,4 I reduzierter Wasserverbrauch gegenüber dem bislang gemäß dem Stand der Technik durchgeführten Abkühlungs- und Trocknungsverfahrens.

| **Parameter** | **Vergleichsbeispiel (Universalprogramm mit Dampfkondensator gemäß** DE 10 2009 013 662 A1**)** | **Ausführungsbeispiel der vorliegenden Erfindung** | **Differenz** |
|---|---|---|---|
| Zeit für Abkühlung (Zeitdifferenz zwischen dem Ende des Abpumpens der Reinigungsflüssigkeit und dem Start des Gebläses zum Austragen der Abluft aus der Reinigungskammer) | 15:00 Minuten | 3:50 Minuten | 11 Minuten |
| Temperatur in der Reinigungskammer zum Startzeitpunkt der Trocknung | 51,4 °C | 62,5 °C (austretende Abluft stets unter 48,8 °C) | 11 °C wärmer, aber deutlich trockenere Luft, die leichtere Trocknung des Reinigungsguts zulässt |
| Wasserverbrauch beim Abkühlen | 2,5l | 1,13 l | 1,4l |

## Patentansprüche

1. Verfahren zum Abkühlen von Abluft eines Reinigungsgeräts, **gekennzeichnet durch** die folgenden Schritte:
a) Einleiten eines ersten Volumens Wasser (20) in einen Dampfkondensator (1) gemäss Anspruch 10 eines Reinigungsgeräts, um eine Dampfsperre gegenüber einem Abluftauslass (8) des Dampfkondensators (1) aufzubauen,
b) Leiten von Abluft (6) aus einer Reinigungskammer des Reinigungsgeräts durch die aus dem Wasser (20) gebildete Dampfsperre hindurch zum Abluftauslass (8), wobei die Abluft (6) in direkten Kontakt mit dem Wasser (20) tritt.

2. Verfahren nach Anspruch 1, **dadurch gekennzeichnet, dass** das Leiten der Abluft (6) aus der Reinigungskammer des Reinigungsgeräts durch die Dampfsperre hindurch dadurch erfolgt, dass ein Gebläse aktiviert wird, welches die Abluft (6) aus der Reinigungskammer zum Dampfkondensator (1) leitet.

3. Verfahren nach Anspruch 1 oder 2, **dadurch gekennzeichnet, dass** das erste Volumen Wasser (20) aus dem Dampfkondensator (1) entfernt wird, nachdem ein vorbestimmbares Volumen der Abluft (6) durch das Wasser (20) hindurch geleitet wurde oder nachdem das erste Volumen Wasser (20) eine Temperatur erreicht hat, die über einem vorbestimmbaren Schwellenwert liegt, oder nachdem die durch das Wasser (20) durchgeleitete Abluft (6) eine Temperatur aufweist, die über einem vorbestimmbaren Schwellenwert liegt.

4. Verfahren nach Anspruch 3, **dadurch gekennzeichnet, dass** ein zweites Volumen Wasser (20) in den Dampfkondensator (1) eingeleitet wird, um eine Dampfsperre gegenüber dem Abluftauslass (8) des Dampfkondensators (1) aufzubauen, nachdem das erste Volumen Wasser (20) aus dem Dampfkondensator (1) entfernt wurde, und dass Schritt b) wiederholt wird.

5. Verfahren nach Anspruch 4, **dadurch gekennzeichnet, dass** das zweite Volumen Wasser (20) aus dem Dampfkondensator (1) entfernt wird, nachdem ein vorbestimmbares Volumen (6) der Abluft durch das Wasser (20) hindurch geleitet wurde oder nachdem das zweite Volumen Wasser (20) eine Temperatur erreicht hat, die über einem vorbestimmbaren Schwellenwert liegt, oder nachdem die durch das Wasser (20) durchgeleitete Abluft (6) eine Temperatur aufweist, die über einem vorbestimmbaren Schwellenwert liegt.

6. Verfahren nach einem der vorherigen Ansprüche, **dadurch gekennzeichnet, dass** ein drittes Volumen Wasser (200) in den Dampfkondensator eingeleitet wird, um eine Dampfsperre gegenüber einer Wasserversorgung (13) für die Reinigungskammer des Reinigungsgeräts aufzubauen, nachdem ein vorheriges Volumen Wasser (20) aus dem Dampfkondensator (1) entfernt wurde, wobei das dritte Volumen Wasser (200) nicht dazu geeignet ist, eine Dampfsperre gegenüber dem Abluftauslass (8) aus dem Dampfkondensator (1) aufzubauen.

7. Verfahren nach einem der vorherigen Ansprüche, **dadurch gekennzeichnet, dass** die Abluft (6) gepulst durch die aus dem Wasser (20) gebildete Dampfsperre hindurch zum Abluftauslass (8) geleitet wird.

8. Verfahren nach einem der vorherigen Ansprüche, **dadurch gekennzeichnet, dass** die Abluft (6) vor oder in der aus dem Wasser (20) gebildeten Dampfsperre durch eine Verteileinrichtung geführt wird, die für eine gleichmäßige Verteilung der Abluft (6) über eine definierte Fläche sorgt.

9. Verfahren nach einem der vorherigen Ansprüche, **dadurch gekennzeichnet, dass** das Volumen des eingeleiteten Wassers (20, 200) mittels eines Sensors oder mittels Vorgabe einer Einlaufzeit bestimmt wird.

10. Dampfkondensator für ein Reinigungsgerät, mit einem Strömungsbereich für Abluft (6) aus einem Reinigungsgerät, einem an einem Ende des Strömungsbereichs angeordneten Abluftauslass (8) und einem Wassereinlass (9), durch den Wasser (10) in den Dampfkondensator (1) geleitet werden kann,
**dadurch gekennzeichnet,**
**dass** der Strömungsbereich derart geformt ist, dass zu dem Abluftauslass (8) strömende Abluft (6) einen Abschnitt des Strömungsbereichs durchströmen muss, der im bestimmungsgemäßen Betrieb des Dampfkondensators (1) vollständig mit Wasser (20) gefüllt ist, und dabei in direkten Kontakt mit dem Wasser (20) tritt.

11. Dampfkondensator nach Anspruch 10, **dadurch gekennzeichnet, dass** im Strömungsbereich eine Barriere (7) derart angeordnet ist, dass im Strömungsbereich befindliches Wasser (20) nicht von einer ersten Seite der Barriere (7) auf eine zweite Seite der Barriere (7) gelangen kann.

12. Dampfkondensator nach Anspruch 10, **dadurch gekennzeichnet, dass** der Wassereinlass (9) zusammen mit einer Wasserversorgung (13) für ein mit dem Dampfkondensator verbundenes Reinigungsgerät in einem gemeinsamen Bauraum (15) des Dampfkondensators (1) angeordnet ist, der mit dem Strömungsbereich in Strömungsverbindung steht.

13. Dampfkondensator nach Anspruch 10 oder 11, **dadurch gekennzeichnet, dass** in dem Abschnitt des Strömungsbereichs, der im bestimmungsgemäßen Betrieb des Dampfkondensators (1) vollständig mit Wasser gefüllt ist, eine Verteileinrichtung für eine gleichmäßige Verteilung von Abluft (6) über eine definierte Fläche angeordnet ist.

14. Reinigungsgerät, **gekennzeichnet durch** einen Dampfkondensator (1) nach einem der Ansprüche 10 bis 13.

## Claims

1. A method for cooling exhaust air of a cleaning device, **characterized by** the following steps:
a) introducing a first volume of water (20) into a steam condenser (1) according to claim 10 of a cleaning device in order to build up a steam barrier with respect to an exhaust air outlet (8) of the steam condenser (1),
b) passing exhaust air (6) from a cleaning chamber of the cleaning device through the steam barrier formed from the water (20), wherein the exhaust air (6) gets in direct contact with the water (20).

2. The method according to claim 1, **characterized in that** passing the exhaust air (6) from the cleaning chamber of the cleaning device through the steam barrier is effected **in that** a blower is activated which conducts the exhaust air (6) from the cleaning chamber to the steam condenser (1).

3. The method according to claim 1 or 2, **characterized in that** the first volume of water (20) is removed from the steam condenser (1) after a predeterminable volume of the exhaust air (6) has been passed through the water (20) or after the first volume of water (20) has reached a temperature that lies above a predeterminable threshold value, or after the exhaust air (6) passed through the water (20) has a temperature that lies above a predeterminable threshold value.

4. The method according to claim 3, **characterized in that** a second volume of water (20) is introduced into the steam condenser (1) in order to build up a steam barrier with respect to the exhaust air outlet (8) of the steam condenser (1) after the first volume of water (20) has been removed from the steam condenser (1), and that step b) is repeated.

5. The method according to claim 4, **characterized in that** the second volume of water (20) is removed from the steam condenser (1) after a predeterminable volume (6) of the exhaust air has been passed through the water (20) or after the second volume of water (20) has reached a temperature that lies above a predeterminable threshold value, or after the exhaust air (6) passed through the water (20) has a temperature that lies above a predeterminable threshold value.

6. The method according to any of the preceding claims, **characterized in that** a third volume of water (200) is introduced into the steam condenser in order to build up a steam barrier with respect to a water supply (13) for the cleaning chamber of the cleaning device, after a previous volume of water (20) has been removed from the steam condenser (1), wherein the third volume of water (200) is not suited to build up a steam barrier with respect to the exhaust air outlet (8) from the steam condenser (1).

7. The method according to any of the preceding claims, **characterized in that** the exhaust air (6) is passed through the steam barrier formed from the water (20) to the exhaust air outlet (8) in a pulsed manner.

8. The method according to any of the preceding claims, **characterized in that** before or in the steam barrier formed from the water (20) the exhaust air (6) is guided through a distributing device that provides for a uniform distribution of the exhaust air (6) over a defined area.

9. The method according to any of the preceding claims, **characterized in that** the volume of the introduced water (20, 200) is determined by means of a sensor or by specifying a run-in period.

10. A steam condenser for a cleaning device, comprising a flow region for exhaust air (6) from a cleaning device, an exhaust air outlet (8) arranged at one end of the flow region, and a water inlet (9) through which water (10) can be introduced into the steam condenser (1),
**characterized in**
**that** the flow region is shaped such that exhaust air (6) flowing to the exhaust air outlet (8) must flow through a portion of the flow region, which in proper operation of the steam condenser (1) is completely filled with water (20), and in doing so gets in direct contact with the water (20).

11. The steam condenser according to claim 10, **characterized in that** in the flow region a barrier (7) is arranged such that water (20) present in the flow region cannot get from a first side of the barrier (7) to a second side of the barrier (7).

12. The steam condenser according to claim 10, **characterized in that** the water inlet (9) together with a water supply (13) for a cleaning device connected to the steam condenser is arranged in a common installation space (15) of the steam condenser (1), which is in flow connection with the flow region.

13. The steam condenser according to claim 10 or 11, **characterized in that** in the portion of the flow region which in proper operation of the steam condenser (1) is completely filled with water a distributing device is arranged for a uniform distribution of exhaust air (6) over a defined area.

14. A cleaning device, **characterized by** a steam condenser (1) according to any of claims 10 to 13.

## Revendications

1. Procédé servant à refroidir de l'air vicié d'un appareil de nettoyage, **caractérisé par** les étapes suivantes :
a) d'introduction d'un premier volume d'eau (20) dans un condensateur de vapeur (1) selon la revendication 10 d'un appareil de nettoyage, pour créer une barrière pare-vapeur par rapport à une sortie d'air vicié (8) du condensateur de vapeur (1),
b) de guidage de l'air vicié (6) hors d'une chambre de nettoyage de l'appareil de nettoyage vers la sortie d'air vicié (8) en traversant de part en part la barrière pare-vapeur formée à partir de l'eau (20), dans lequel l'air vicié (6) entre en contact direct avec l'eau (20).

2. Procédé selon la revendication 1, **caractérisé en ce que** le guidage de l'air vicié (6) hors de la chambre de nettoyage de l'appareil de nettoyage en traversant la barrière pare-vapeur de part en part est effectué **en ce qu'**une soufflante est activée, laquelle guide l'air vicié (6) hors de la chambre de nettoyage vers le condensateur de vapeur (1).

3. Procédé selon la revendication 1 ou 2, **caractérisé en ce que** le premier volume d'eau (20) est retiré du condensateur de vapeur (1) après qu'un volume pouvant être prédéfini de l'air vicié (6) a été guidé à travers l'eau (20) ou après que le premier volume d'eau (20) a atteint une température, qui est supérieure à une valeur de seuil pouvant être prédéfinie ou après que l'air vicié (6) traversé par l'eau (20) présente une température, qui est supérieure à la valeur de seuil pouvant être prédéfinie.

4. Procédé selon la revendication 3, **caractérisé en ce qu'**un deuxième volume d'eau (20) est introduit dans le condensateur à vapeur (1), pour créer une barrière pare-vapeur par rapport à la sortie d'air vicié (8) du condensateur de vapeur (1), après que le premier volume d'eau (20) a été retiré du condensateur à vapeur (1) et que l'étape b) est répétée.

5. Procédé selon la revendication 4, **caractérisé en ce que** le deuxième volume d'eau (20) est retiré du condensateur de vapeur (1) après qu'un volume pouvant être prédéfinie de l'air vicié (6) a été guidé à travers l'eau (20) de part en part ou après que le deuxième volume d'eau (20) a atteint une température, qui est supérieure à une valeur de seuil pouvant être prédéfinie ou après que l'air vicié (6) guidé à travers l'eau (20) de part en part présente une température, qui est supérieure à une valeur de seuil pouvant être prédéfinie.

6. Procédé selon l'une quelconque des revendications précédentes, **caractérisé en ce qu'**un troisième volume d'eau (200) est introduit dans le condensateur de vapeur pour créer une barrière pare-vapeur par rapport à une alimentation en eau (13) pour la chambre de nettoyage de l'appareil de nettoyage, après qu'un volume d'eau (20) précédent a été retiré du condensateur de vapeur (1), dans lequel le troisième volume d'eau (200) n'est pas adapté pour créer une barrière pare-vapeur par rapport à la sortie d'air vicié (8) hors du condensateur de vapeur (1).

7. Procédé selon l'une quelconque des revendications précédentes, **caractérisé en ce que** l'air vicié (6) est guidé de manière pulsée à travers la barrière pare-vapeur formée à partir de l'eau (20) de part en part vers la sortie d'air vicié (8).

8. Procédé selon l'une quelconque des revendications précédentes, **caractérisé en ce que** l'air vicié (6) est conduit par un dispositif de répartition avant ou dans la barrière pare-feu formée à partir de l'eau (20), qui veille à une répartition homogène de l'air vicié (6) sur une surface définie.

9. Procédé selon l'une quelconque des revendications précédentes, **caractérisé en ce que** le volume de l'eau (20, 200) introduite est défini au moyen d'un capteur ou au moyen d'une spécification d'une période de rodage.

10. Condensateur de vapeur pour un appareil de nettoyage, avec une zone d'écoulement pour de l'air vicié (6) hors d'un appareil de nettoyage, une sortie d'air vicié (8) disposée au niveau d'une extrémité de la zone d'écoulement et une entrée d'eau (9), par laquelle de l'eau (10) peut être guidée dans le condensateur de vapeur (1),
**caractérisé en ce**
**que** la zone d'écoulement est formée de telle manière que de l'air vicié (6) s'écoulant vers la sortie d'air vicié (8) doit traverser une section de la zone d'écoulement, qui est remplie, lors du fonctionnement en bonne et due forme du condensateur de vapeur (1), en totalité d'eau (20) et entre ce faisant en contact direct avec l'eau (20).

11. Condensateur de vapeur selon la revendication 10, **caractérisé en ce qu'**une barrière (7) est disposée dans la zone d'écoulement de telle manière que de l'eau (20) se trouvant dans la zone d'écoulement ne peut parvenir depuis un premier côté de la barrière (7) sur un deuxième côté de la barrière (7).

12. Condensateur de vapeur selon la revendication 10, **caractérisé en ce que** l'entrée d'eau (9) est disposée, conjointement avec une alimentation en eau (13) pour un appareil de nettoyage relié au condensateur de vapeur, dans un espace de montage (15) commun du condensateur de vapeur (1), qui est en communication fluidique avec la zone d'écoulement.

13. Condensateur de vapeur selon la revendication 10 ou 11, **caractérisé en ce qu'**un dispositif de répartition pour une répartition homogène de l'air vicié (6) sur une surface définie est disposé dans la section de la zone d'écoulement, qui est remplie lors du fonctionnement en bonne et due forme du condensateur de vapeur (1) en totalité d'eau.

14. Appareil de nettoyage, **caractérisé par** un condensateur de vapeur (1) selon l'une quelconque des revendications 10 à 13.
